(19)

Europäisches Patentamt
European Patent Office
Office européen des brevets

(11) **EP 3 103 815 B1**

(12) **EUROPEAN PATENT SPECIFICATION**

(45) Date of publication and mention of the grant of the patent:
**19.12.2018 Bulletin 2018/51**

(21) Application number: **15737598.1**

(22) Date of filing: **13.01.2015**

(51) Int Cl.:
*C08B 37/08* (2006.01)          *A61K 8/73* (2006.01)
*A61K 31/728* (2006.01)          *A61Q 19/00* (2006.01)
*A61Q 19/02* (2006.01)          *A61K 9/00* (2006.01)
*A61K 47/36* (2006.01)          *A61K 9/20* (2006.01)
*A61K 9/48* (2006.01)          *A23L 33/10* (2016.01)

(86) International application number:
**PCT/JP2015/050638**

(87) International publication number:
**WO 2015/108029 (23.07.2015 Gazette 2015/29)**

(54) **HYALURONIC ACID AND/OR SALT THEREOF, METHOD FOR PRODUCING SAME, AND FOOD, COSMETIC, AND PHARMACEUTICAL CONTAINING SAID HYALURONIC ACID AND/OR SALT THEREOF**

HYALURONSÄURE UND/ODER SALZ DAVON, VERFAHREN ZUR HERSTELLUNG DAVON UND NAHRUNGSMITTEL, KOSMETIKUM UND ARZNEIMITTEL MIT DER HYALURONSÄURE UND/ODER DEM SALZ DAVON

ACIDE HYALURONIQUE ET/OU SON SEL, LEUR PROCEDE DE PRODUCTION, ET ALIMENT, PRODUIT COSMETIQUE ET PHARMACEUTIQUE CONTENANT LEDIT ACIDE HYALURONIQUE ET/OU SON SEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.01.2014  JP 2014004677**
**09.04.2014  JP 2014080566**

(43) Date of publication of application:
**14.12.2016  Bulletin 2016/50**

(73) Proprietor: **Kewpie Corporation**
**Tokyo 150-0002 (JP)**

(72) Inventors:
• **YAMAZAKI, Taro**
**Chofu-shi**
**Tokyo 182-0002 (JP)**

• **KAJIYAMA, Daichi**
**Chofu-shi**
**Tokyo 182-0002 (JP)**

(74) Representative: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) References cited:
| EP-A1- 1 865 002 | EP-A1- 1 992 645 |
| EP-A1- 2 511 302 | WO-A1-2008/004530 |
| WO-A1-2011/070948 | JP-A- H02 142 801 |
| JP-A- S62 289 197 | JP-A- 2009 155 486 |
| JP-A- 2012 001 696 | JP-A- 2013 177 608 |

**Description**

Technical Field

The present invention relates to a hyaluronic acid and/or a salt thereof, a production method therefor, and a food, a cosmetic, and a pharmaceutical containing the hyaluronic acid and/or the salt thereof.

Background Art

Hyaluronic acid is widely distributed in body tissues, such as a cockscomb, an umbilical cord, skin, cartilage, a vitreous body, and synovial fluid, and is widely used as, for example, an ingredient of a cosmetic, a pharmaceutical, or a food. When the hyaluronic acid is used as an ingredient of a cosmetic, a pharmaceutical, or a food, the hyaluronic acid is required to have handling ability such as tableting adequacy in formulation.

Purification of the hyaluronic acid is typically carried out through precipitation by addition of ethanol or through freeze-drying (JP 2006-265287 A, JP 2009-155486 A and EP 2 511 302 A1).

However, when the hyaluronic acid is purified through freeze-drying, typically, a hyaluronic acid in a solid state (for example, a cotton-like product) having a low density is obtained. Such hyaluronic acid having a low density may be inferior in tableting adequacy in formulation.

In addition, when a hyaluronic acid having a lower molecular weight is purified by precipitation through addition of an organic solvent, such as ethanol, methanol, acetone, isopropanol, or hexane, the hyaluronic acid is liable to be hardly formed as a precipitate in the organic solvent, and hence the amount of a hyaluronic acid to be purified may be reduced.

JP 2013 177608 A discloses a method for preparing hyaluronic acid wherein hyaluronic acid having an average molecular weight of from 2,000,000 to 8,000,000 is acid hydrolyzed and the resulting hydrolysate is purified by desalting. EP 1 865 002 A1 and EP 1 992 645 A1 disclose methods for preparing hyaluronic acid wherein hyaluronic acid having an average molecular weight of from 2,000,000 to 8,000,000 is acid hydrolyzed in suspension and the resulting hydrolysate is purified by precipitation.

Summary of Invention

Technical Problem

The present invention provides a hyaluronic acid and/or a salt thereof, which is excellent in handling ability, such as tableting adequacy, a method of producing the hyaluronic acid and/or the salt thereof, and a food, a cosmetic, and a pharmaceutical each containing the hyaluronic acid and/or the salt thereof.

Solution to Problem

The inventors of the present application have found that a hyaluronic acid and/or a salt thereof having a predetermined molecular weight and a predetermined bulk density has excellent properties (for example, handling ability) and can be prepared by the following methods.

  1. A method of producing a hyaluronic acid and/or a salt thereof according to one embodiment of the present invention includes:

        preparing an acidic raw material hyaluronic acid solution that includes an alcohol having 1, 2 or 3 carbon atoms, a raw material hyaluronic acid and/or a salt thereof having an average molecular weight of 3,500 or more and 100,000 or less, and an acid which can adjust the pH of the acidic raw material hyaluronic acid solution to an acidic pH;
        subjecting the raw material hyaluronic acid and/or the salt thereof to acid hydrolysis in the acidic raw material hyaluronic acid solution obtained in the preparing;
        treating an acidic solution that includes a hydrolysate obtained through the acid hydrolysis with an anion-exchange resin; and
        subjecting the hydrolysate treated with the resin to spray-drying or freeze-drying,
        the acidic raw material hyaluronic acid solution including 15 mass% or more and 35 mass% or less of the raw material hyaluronic acid and/or the salt thereof,
        the acidic raw material hyaluronic acid solution including 5 parts by mass or more and 60 parts by mass or less of the alcohol having 1, 2 or 3 carbon atoms with respect to 100 parts by mass of the raw material hyaluronic acid and/or the salt thereof.

2. In the method of producing a hyaluronic acid and/or a salt thereof according to Item 1, the spray-drying may include subjecting a liquid including the hydrolysate to spray-drying, and a mass of the hydrolysate with respect to a mass of the liquid including the hydrolysate may be 8 mass% or more and 35 mass% or less.

Advantageous Effects of Invention

The hyaluronic acid and/or the salt thereof obtained by the method according to any one of Items 1 and 2 has an average molecular weight of 3,500 or less, a proportion of disaccharide of 1 mass% or more and 30 mass% or less, a content of chlorine of 1 mass% or less, a content of sulfur of 1 mass% or less, and a bulk density of more than 0.25 $g/cm^3$, and hence is excellent in handling ability, such as tableting adequacy. The hyaluronic acid and/or the salt thereof can be used as an ingredient of, for example, a food, a cosmetic, or a pharmaceutical.

The method of producing a hyaluronic acid and/or a salt thereof according to Item 1 or 2 includes: preparing an acidic raw material hyaluronic acid solution that includes a lower alcohol, a raw material hyaluronic acid and/or a salt thereof having an average molecular weight of 3,500 or more and 100,000 or less, and an acid; subjecting the raw material hyaluronic acid and/or the salt thereof to acid hydrolysis in the acidic raw material hyaluronic acid solution obtained in the preparing; treating an acidic solution that includes a hydrolysate obtained through the acid hydrolysis with an anion-exchange resin; and subjecting the hydrolysate treated with the resin to spray-drying or freeze-drying, the acidic raw material hyaluronic acid solution including 15 mass% or more and 35 mass% or less of the raw material hyaluronic acid and/or the salt thereof, the acidic raw material hyaluronic acid solution including 5 parts by mass or more and 60 parts by mass or less of the lower alcohol with respect to 100 parts by mass of the raw material hyaluronic acid and/or the salt thereof, and hence the hyaluronic acid and/or the salt thereof having an average molecular weight of 3,500 or less, a proportion of disaccharide of 1 mass% or more and 30 mass% or less, a content of chlorine of 1 mass% or less, a content of sulfur of 1 mass% or less, and a bulk density of more than 0.25 $g/cm^3$ can be efficiently produced.

Description of Embodiments

The present invention is hereinafter described in detail. In the present invention, "part(s)" means "part(s) by mass" and "%" means "mass%" unless otherwise specified.

[Hyaluronic Acid and/or Salt Thereof]

A hyaluronic acid and/or a salt thereof obtained by the method of the present invention has an average molecular weight of 3,500 or less, a proportion of disaccharide of 1 mass% or more and 30 mass% or less, a content of chlorine of 1 mass% or less, a content of sulfur of 1 mass% or less, and a bulk density of more than 0.25 $g/cm^3$.

<Definition of Hyaluronic Acid>

In the present invention, the term "hyaluronic acid" refers to a polysaccharide having at least one repeating structural unit consisting of a disaccharide of a glucuronic acid and N-acetylglucosamine. In addition, the "salt of hyaluronic acid" is not particularly limited, and is preferably an acceptable salt in food or pharmacy. Examples thereof include a sodium salt, a potassium salt, a calcium salt, a zinc salt, a magnesium salt, and an ammonium salt.

A hyaluronic acid is basically a disaccharide or a polysaccharide which includes at least one disaccharide unit in which a C-1 position of β-D-glucuronic acid and a C-3 position of β-D-N-acetyl-glucosamine are bonded to each other, is basically formed of β-D-glucuronic acid and β-D-N-acetyl-glucosamine, and has one or a plurality of the disaccharide units bonded to each other. The hyaluronic acid and/or the salt thereof may be substantially free of a non-reducing end sugar (for example, a sugar having an unsaturated bond between a C-4 position and a C-5 position of a glucuronic acid) (that is, a content of the non-reducing end sugar may be 1 mass% or less).

In the present invention, the term "disaccharide" refers to one disaccharide unit (consisting of two saccharides) in which a C-1 position of β-D-glucuronic acid and a C-3 position of β-D-N-acetyl-glucosamine are bonded to each other, the term "tetrasaccharide" refers to two disaccharide units (consisting of four saccharides), the term "hexasaccharide" refers to three disaccharide units (consisting of six saccharides), and the term "octasaccharide" refers to four disaccharide units (consisting of eight saccharides) . In the present invention, all of the terms "disaccharide," "tetrasaccharide," "hexasaccharide," and "octasaccharide" refer to saccharides including no saccharide having a non-reducing end (saturated sugars).

(Bulk Density)

In the present invention, the "bulk density" is a value measured in conformity with "the Japanese Pharmacopoeia (16th edition), 3. Powder Property Determinations, 3.01 Determination of Bulk and Tapped Densities." The hyaluronic acid and/or the salt thereof has an average molecular weight of 3,500 or less and a bulk density of more than 0.25 g/cm$^3$, and hence has a low molecular weight but is excellent in handling ability, such as tableting adequacy.

(Average Molecular Weight)

When the hyaluronic acid and/or the salt thereof has an average molecular weight of 3,500 or less, preferably 2,500 or less, and also has an average molecular weight of typically 800 or more, preferably 1, 000 or more, a higher purity can be achieved. The average molecular weight of the hyaluronic acid and/or the salt thereof may be measured by a method using HPLC to be described later.

More specifically, values are calculated by multiplying molecular weights (for example, in the case of disaccharide, molecular weight: 400) of peaks of the hyaluronic acid and/or the salt thereof determined by HPLC measurement by peak areas, and the values calculated for the respective peaks are integrated. Further, the integrated value was divided by a total peak area to calculate an average molecular weight.

(Purity)

The hyaluronic acid and/or the salt thereof has a purity of 90% or more, preferably 95% or more, more preferably 98% or more.

In the present invention, the term "purity" refers to a proportion of ingredients other than ingredients excluding a hyaluronic acid and/or a salt thereof contained in the hyaluronic acid and/or the salt thereof. As the ingredients excluding the hyaluronic acid and/or the salt thereof, there are given, for example, monosaccharides (for example, glucuronic acid, N-acetylglucosamine, and a monosaccharide derived therefrom) and a non-reducing end sugar.

The purity of the hyaluronic acid and/or the salt thereof may be calculated by subtracting peaks corresponding to monosaccharides from HPLC spectrum data of the hyaluronic acid and/or the salt thereof. In the hyaluronic acid and/or the salt thereof, the proportion of the monosaccharides is typically 3 mass% or less, preferably 2 mass% or less.

(Degree of Whiteness)

The hyaluronic acid and/or the salt thereof may have a b value indicating the hue of a color (hereinafter sometimes referred to simply as "b value") of 10 or less, preferably 8 or less, more preferably 5 or less, and generally has a b value of 0 or more.

The b value is a value specifying the hue of the color of a substance. As the b value becomes larger, the degree of yellowness becomes higher. In contrast, as the b value becomes smaller, the degree of blueness becomes higher.

The low molecular hyaluronic acid and/or the salt thereof has an L value indicating lightness of a color (hereinafter sometimes referred to simply as "L value") of 90 or more, and has an L value of preferably 92 or more, more preferably 93 or more.

The L value is a value specifying the lightness of the color of a substance, and is expressed as a numerical value between 0 and 100. An L value of 100 indicates the brightest state (completely white), and an L value of 0 indicates the darkest state (completely black).

The b value and the L value may each be indicated by Lab chromaticity coordinates according to a color difference indication method specified in JIS Z 8730-1995. In addition, the b value and the L value may each be measured using a commercially-available color difference meter. In the present invention, the b value and the L value of the low molecular hyaluronic acid and/or the salt thereof in a solid state are measured.

The b value and the L value of the hyaluronic acid and/or the salt thereof may each be measured with, for example, a color difference meter (trade name "Color And Color Difference Meter Model 1001 DP" manufactured by Nippon Denshoku Industries Co., Ltd.) having mounted thereon a 10 φ lens and having a glass cell charged with 1 g or more of a measurement sample.

When the hyaluronic acid and/or the salt thereof has a b value indicating the hue of a color measured with a color difference meter of 10 or less (further, an L value of 90 or more), the hyaluronic acid and/or the salt thereof can be used as a raw material for, for example, cosmetics, foods, and pharmaceuticals because of having a high degree of whiteness.

(Content of Sulfur and Content of Chlorine)

The hyaluronic acid and/or the salt thereof has a content of chlorine of 1 mass% or less and a content of sulfur of 1 mass% or less, and hence is excellent in tableting adequacy. When the content of chlorine or sulfur in the hyaluronic acid and/or the salt thereof is more than 1 mass%, it is supposed that the amount of a metal salt of chlorine, such as sodium chloride, or a metal salt of sulfate, such as sodium sulfate, may increase to raise a degree of non-uniformity of particle shapes, resulting in decreasing a binding strength in tableting. Meanwhile, the hyaluronic acid and/or the salt thereof has a content of chlorine of 1 mass% or less and a content of sulfur of 1 mass% or less, and the content of chlorine and the content of sulfur are low. Therefore, the uniformity of particle shapes increases to enhance the binding strength in tableting, resulting in improving tableting adequacy.

The hyaluronic acid and/or the salt thereof obtained by the method of the present invention can be used in a food, a pharmaceutical, or a cosmetic.

For example, a salt, such as sodium chloride, may impair stability of a system. In particular, in an emulsification system, the salt may cause demulsification. From a viewpoint of being directly applied to the skin or hair or being ingested, and hence the amount of chloride or sulfur derived from a halide, such as sodium chloride, a sulfate such as sodium sulfate, or an acid such as hydrochloric acid or sulfuric acid, is desirably reduced as much as possible.

Accordingly, when the hyaluronic acid and/or the salt thereof may have a content of chlorine of 1 mass% or less, and has a content of chlorine of preferably 0.8 mass% or less, more preferably 0.5 mass% or less, a high bulk density, high emulsification stability, and high safety can be achieved.

The content of chlorine and the content of sulfur in the hyaluronic acid and/or the salt thereof may be measured by a known method (for example, an element analysis (combustion method, ICP emission spectrometric analysis method, atomic absorption method, fluorescent X-ray analysis method), mass spectrometry) . Further, the content of chlorine of the hyaluronic acid and/or the salt thereof may be calculated from a value determined by Mohr's method.

(Average Particle Diameter)

When the hyaluronic acid and/or the salt thereof may have an average particle diameter of 10 μm or more and 35 μm or less, and has an average particle diameter of preferably 15 μm or more and preferably 30 μm or less, excellent tableting adequacy can be achieved.

(Standard Deviation of Particle Diameters)

The standard deviation of particle diameters is an indicator of a variation of particle diameters of the hyaluronic acid and/or the salt thereof. When the standard deviation of particle diameters of the hyaluronic acid and/or the salt thereof may be 0.1 or more and 0.3 or less, and is preferably 0.15 or more and preferably 0.25 or less, a small variation of particle diameters can increase a density, resulting in improving the tableting adequacy.

(Proportion of Disaccharide)

When the hyaluronic acid and/or the salt thereof has a proportion of disaccharide of 1 mass% or more and 30 mass% or less, and has a proportion of disaccharide of preferably 5 mass% or more, more preferably 8 mass% or more and preferably 25 mass% or less, more preferably 20 mass% or less, the hyaluronic acid and/or the salt thereof can exhibit original physiological functions of the hyaluronic acid.

When the molecular weight of a hyaluronic acid and/or a salt thereof is reduced with an animal-derived or microorganism-derived enzyme (hyaluronic acid-degrading enzyme), typically, disaccharide (saturated disaccharide) is hardly obtained. Meanwhile, when the production method of the invention is adopted, the hyaluronic acid and/or the salt thereof containing disaccharide can be obtained.

The hyaluronic acid and/or the salt thereof has a proportion of disaccharide of 1 mass% or more and 30 mass% or less, and hence contains a predetermined amount of sugars each having a short sugar chain, which enhances a hygroscopic property and increases a binding strength, and hence the hyaluronic acid and/or the salt thereof can be obtained as bulky particles. In addition, the hyaluronic acid and/or the salt thereof may have a proportion of monosaccharide of 1.5 mass% or more and 3 mass% or less.

(Hyaluronic Acid-degrading Enzyme)

When the hyaluronic acid and/or the salt thereof may be free of a hyaluronic acid-degrading enzyme, antigenicity can be reduced. In the present invention, the phrase "free of a hyaluronic acid-degrading enzyme" refers to the hyaluronic acid and/or the salt thereof having a content of the hyaluronic acid-degrading enzyme of 1 mass% or less.

When the molecular weight of a hyaluronic acid and/or a salt thereof is reduced through the use of an enzyme (hyaluronidase), the hyaluronic acid-degrading enzyme may remain in the resultant hyaluronic acid and/or the salt thereof having a reduced molecular weight.

Meanwhile, the hyaluronic acid and/or the salt thereof obtained by the production method of the invention without using the hyaluronic acid-degrading enzyme contains no hyaluronic acid-degrading enzyme, and hence has reduced antigenicity.

(Proportion of Octasaccharide)

When the hyaluronic acid and/or the salt thereof may have a proportion of octasaccharide of 1 mass% or more and 30 mass% or less, and has a proportion of octasaccharide of more preferably 5 mass% or more, the hyaluronic acid and/or the salt thereof can more surely exhibit physiological activities derived from octasaccharide.

(Total of Disaccharide, Tetrasaccharide, Hexasaccharide, and Octasaccharide)

The hyaluronic acid and/or the salt thereof may have a total proportion of disaccharide, tetrasaccharide, hexasaccharide, and octasaccharide of 5 mass% or more and 80 mass% or less, and has a total proportion of disaccharide, tetrasaccharide, hexasaccharide, and octasaccharide of preferably 10 mass% or more and preferably 70 mass% or less.

For example, the hyaluronic acid and/or the salt thereof may have an average molecular weight of 3,500 or less and a total proportion of disaccharide, tetrasaccharide, hexasaccharide, and octasaccharide of 5 mass% or more and 80 mass% or less, or 20 mass% or more and 60 mass% or less. In this case, the proportion of disaccharide may be 1 mass% or more and 30 mass% or less. In addition, in this case, the proportion of octasaccharide may be 1 mass% or more and 30 mass% or less.

The proportions (mass%) of disaccharide, tetrasaccharide, hexasaccharide, and octasaccharide in the hyaluronic acid and/or the salt thereof may be determined by HPLC for the hyaluronic acid and/or the salt thereof. More specifically, the proportions (mass%) of disaccharide, tetrasaccharide, hexasaccharide, and octasaccharide in the hyaluronic acid and/or the salt thereof may be calculated by dividing peak areas of peaks (for example, in the case of disaccharide, molecular weight: 400) of the hyaluronic acid and/or the salt thereof determined by HPLC measurement by a total peak area.

(Kinetic Viscosity)

A 5 mass% aqueous solution of the hyaluronic acid and/or the salt thereof may have a kinetic viscosity of 2.0 mm$^2$/s or less (preferably 1.5 mm$^2$/s or less, more preferably 1.3 mm$^2$/s or less) .

The kinetic viscosity of the 5 mass% aqueous solution of the hyaluronic acid and/or the salt thereof may be measured using an Ubbelohde viscometer (manufactured by Sibata Scientific Technology Ltd.). In this case, an Ubbelohde viscometer having such a coefficient that a falling time is from 200 seconds to 1,000 seconds is selected. Further, the kinetic viscosity is measured in a thermostat bath at 30°C while a constant temperature is maintained. The kinetic viscosity (unit: mm$^2$/s) may be determined by multiplying the falling time of the aqueous solution measured using the Ubbelohde viscometer by the coefficient of the Ubbelohde viscometer.

(Action and Effect)

The hyaluronic acid and/or the salt thereof obtained by the method of the present invention has an average molecular weight of 3,500 or less, a proportion of disaccharide of 1 mass% or more and 30 mass% or less, a content of chlorine of 1 mass% or less, a content of sulfur of 1 mass% or less, and a bulk density of more than 0.25 g/cm$^3$, and hence is excellent in handling ability, such as tableting adequacy.

[Method of Producing Hyaluronic Acid and/or Salt Thereof]

A method of producing a hyaluronic acid and/or a salt thereof according to one embodiment of the present invention (hereinafter sometimes referred to as "first production method") includes: preparing an acidic raw material hyaluronic acid solution containing a lower alcohol, a raw material hyaluronic acid and/or a salt thereof having an average molecular weight of 3,500 or more and 100,000 or less, and an acid which can adjust the pH of the acidic raw material hyaluronic acid to an acidic pH; subjecting the raw material hyaluronic acid and/or the salt thereof to acid hydrolysis in the acidic raw material hyaluronic acid solution obtained in the preparation step (hereinafter sometimes referred to as "liquid phase decomposition step"); treating an acidic solution containing the hydrolysate obtained through the acid hydrolysis with an anion-exchange resin (hereinafter sometimes referred to as "anion-exchange resin treatment step") ;

and a step of subjecting the hydrolysate treated with the resin to spray-drying or freeze-drying (hereinafter sometimes referred to as "spray-drying step" or "freeze-drying step"), and the acidic raw material hyaluronic acid solution contains 15 mass% or more and 35 mass% or less of the raw material hyaluronic acid and/or the salt thereof, and contains 5 parts by mass or more and 60 parts by mass or less of the lower alcohol with respect to 100 parts by mass of the raw material hyaluronic acid and/or the salt thereof.

<Preparing Acidic Raw Material Hyaluronic Acid Solution>

In preparing an acidic raw material hyaluronic acid solution, an acidic raw material hyaluronic acid solution containing a lower alcohol, a raw material hyaluronic acid and/or a salt thereof having an average molecular weight of 3,500 or more and 100,000 or less are used, and an acid is prepared.

(Molecular Weight of Raw Material Hyaluronic Acid and/or Salt Thereof)

In the production method according to the embodiment of the present invention, when the raw material hyaluronic acid and/or the salt thereof contained in the acidic raw material hyaluronic acid solution has a molecular weight of preferably 5,000 or more, more preferably 6,000 or more, much more preferably 8,000 or more and also has a molecular weight of preferably 50,000 or less, more preferably 20,000 or less, a hyaluronic acid and/or a salt thereof having a molecular weight of 3,500 or less and a high degree of whiteness can be obtained.

(Method of Measuring Molecular Weight of (Starting) Raw Material Hyaluronic Acid and/or Salt Thereof)

In the present invention, the average molecular weight of the raw material hyaluronic acid and/or the salt thereof (and the starting raw material hyaluronic acid and/or the salt thereof to be described later) may be measured by the following method. That is, about 0.05 g of hyaluronic acid (original substance) is accurately weighed and dissolved in a 0.2 mol/L sodium chloride solution to prepare exactly 100 mL of the resultant solution. The solution is weighed exactly in amounts of 8 mL, 12 mL, and 16 mL, and a 0.2 mol/L sodium chloride solution is added to the respective solutions to prepare exactly 20 mL of the mixed solutions as sample solutions. The specific viscosity of each of the sample solutions and the 0.2 mol/L sodium chloride solution is measured at 30.0±0.1°C by a viscosity measurement method (first method, capillary viscosity measurement method) of General Tests, Processes and Apparatus of the Japanese Pharmacopoeia (16th edition) (Expression (A)), and the reduced viscosity at each concentration is calculated (Expression (B)). The reduced viscosity and the concentration of the substance calculated on a dried basis (g/100 mL) are plotted in the vertical axis and the horizontal axis, respectively, on a graph, and the limiting viscosity is calculated from the intersection point of a straight line that connects each point and the vertical axis. The determined limiting viscosity is substituted into a Laurent Expression (Expression (C)) to calculate the average molecular weight (Torvard C Laurent, Marion Ryan, and Adolph Pietruszkiewicz, "Fractionation of hyaluronic Acid," Biochemina et Biophysica Acta., 42, 476-485 (1960); Chikako Yomota, "Evaluation of Molecular Weights of Sodium Hyaluronate Preparations by SEC-MALLS," Bull. Natl. Inst. Health Sci., 121, 030-033 (2003)) .

```
(Expression A) Specific viscosity={required number of
seconds for falling of sample solution}/(required number of
seconds for falling of 0.2 mol/L sodium chloride solution)}-1
```

```
(Expression B) Reduced viscosity (dL/g)=specific
viscosity/(concentration of substance calculated on dried basis
(g/100 mL)
```

$$\text{(Expression C) Limiting viscosity (dL/g)}=3.6\times10^{-4}M^{0.78}$$

M: Average molecular weight

(Concentration of Raw Material Hyaluronic Acid and/or Salt)

The acidic raw material hyaluronic acid solution contains the raw material hyaluronic acid and/or the salt thereof prefer ably at a concentration at which the raw material hyaluronic acid and/or the salt thereof is soluble in the acidic raw material hyaluronic acid solution.

More specifically, when the concentration of the raw material hyaluronic acid and/or the salt thereof in the acidic raw material hyaluronic acid solution (the mass of the raw material hyaluronic acid and/or the salt thereof with respect to the mass of the acidic raw material hyaluronic acid solution) is 15 mass% or more, preferably 16 mass% or more, more preferably 18 mass% or more, and is also 35 mass% or less, preferably 32 mass% or less, in the liquid phase decomposition step, browning of the raw material hyaluronic acid and/or the salt thereof can be prevented, a reduction in the molecular weight of the raw material hyaluronic acid and/or the salt thereof (for example, adjustment of the average molecular weight of the hyaluronic acid and/or the salt thereof to be obtained to 3,500 or less) can be achieved, and generation of monosaccharide can be suppressed.

(Solvent)

When a solvent to be used in the acidic raw material hyaluronic acid solution is a water-containing solvent (preferably water), the raw material hyaluronic acid and/or the salt thereof has excellent solubility in the solvent. The solvent may be water or a water-containing solvent containing 30 mass% or less of a water-miscible organic solvent as long as the raw material hyaluronic acid and/or the salt thereof is soluble in the solvent.

(Lower Alcohol)

In the present invention, the term "lower alcohol" refers to an alcohol having 1, 2, or 3 carbon atoms, and examples thereof include methanol, ethanol, 1-propanol, and 2-propanol. Of those, one kind or two or more kinds of the alcohols may be used as the lower alcohol. The lower alcohol is preferably, for example, methanol and/or ethanol, more preferably ethanol.

When the acidic raw material hyaluronic acid solution preferably contains 10 parts by mass or more, or 50 parts by mass or less of the lower alcohol with respect to 100 parts by mass of the raw material hyaluronic acid and/or the salt thereof, the molecular weight of a hyaluronic acid and/or a salt thereof obtained as a final product can be more surely adjusted to 3,500 or less.

(Acid)

The acid contained in the acidic raw material hyaluronic acid solution may be any acid as long as the acid can adjust the pH of the acidic raw material hyaluronic acid solution to an acidic pH. From the viewpoint of reduction of the amount of the acid to be used, as the acid, for example, an inorganic acid, such as hydrochloric acid, sulfuric acid, hydrobromic acid, nitric acid, or phosphoric acid, or an organic acid, such as acetic acid, citric acid, a sulfone group-containing organic acid (for example, paratoluenesulfonic acid or trifluoromethanesulfonic acid), or a fluorine atom-containing organic acid (for example, trifluoroacetic acid) may be used.

(Addition Method)

In preparing the acidic raw material hyaluronic acid solution, the acidic raw material hyaluronic acid solution may be prepared by adding a mixture of the lower alcohol and the raw material hyaluronic acid and/or the salt thereof (in this step, the mixture contains 5 parts by mass or more and 60 parts by mass or less of the lower alcohol with respect to 100 parts by mass of the raw material hyaluronic acid and/or the salt thereof) to a liquid containing an acid (acidic liquid).

Alternatively, the acidic raw material hyaluronic acid solution may also be prepared by adding an acid to the solution containing the raw material hyaluronic acid and/or the salt thereof and the lower alcohol.

When the acidic raw material hyaluronic acid solution is prepared by adding the mixture to the acidic liquid, the raw material hyaluronic acid and/or the salt thereof may be moistened with the lower alcohol in the mixture. In this case, when the acidic raw material hyaluronic acid solution is prepared using the raw material hyaluronic acid and/or the salt thereof obtained in the solid phase decomposition step to be described later, the acidic raw material hyaluronic acid solution may be prepared through decantation after the solid phase decomposition step so that the proportion of the lower alcohol used in the solid phase decomposition step is adjusted as desired.

In the present invention, the phrase "the raw material hyaluronic acid and/or the salt thereof is moistened with the lower alcohol" refers to a state in which at least a part of the raw material hyaluronic acid and/or the salt thereof is present as a solid in the lower alcohol, and the raw material hyaluronic acid and/or the salt thereof in a solid state is wetted and swollen with the lower alcohol.

When the acidic raw material hyaluronic acid solution is prepared by adding the mixture of the raw material hyaluronic acid and/or the salt thereof and the lower alcohol to the acidic liquid under a state in which the raw material hyaluronic acid and/or the salt thereof is moistened with the lower alcohol, aggregation of the raw material hyaluronic acid and/or the salt thereof in the acidic liquid (that is, the raw material hyaluronic acid and/or the salt thereof aggregates in the acidic liquid and is not dispersed uniformly in the acid solution) can be prevented to enhance dispersibility of the raw material hyaluronic acid and/or the salt thereof in the acidic liquid.

When the mass of the acidic liquid before addition of the mixture is defined as 100 parts by mass, the mass of the mixture to be added with respect to the mass of the acidic liquid before addition of the mixture (the mass of the mixture/the mass of the acidic liquid before addition of the mixture) is preferably 10 parts by mass or more and 40 parts by mass or less, more preferably 20 parts by mass or more, and is more preferably 30 parts by mass or less, in this case, the reduction in the molecular weight can be carried out more easily.

(pH of Acidic Raw Material Hyaluronic Acid Solution)

When the acidic raw material hyaluronic acid solution may have a pH of 0.2 or more and 2 or less, and has a pH of preferably 0.3 or more, more preferably 0.5 or more, even more preferably 0.8 or more, and also has a pH of preferably 1.8 or less, more preferably 1.5 or less, even more preferably 1.0 or less, in the liquid phase decomposition step, browning of the raw material hyaluronic acid and/or the salt thereof can be prevented, and a reduction in molecular weight of the raw material hyaluronic acid and/or the salt thereof can be achieved.

<Liquid Phase Decomposition Step>

In the liquid phase decomposition step (first molecular-weight reduction step), a raw material hyaluronic acid and/or a salt thereof having an average molecular weight of 3,500 or more and 100,000 or less is retained in an acidic raw material hyaluronic acid solution containing 15 mass% or more and 35 mass% or less of the raw material hyaluronic acid and/or the salt thereof to subject the raw material hyaluronic acid and/or the salt thereof to acid hydrolysis. Thus, the molecular weight of the raw material hyaluronic acid and/or the salt thereof is reduced through the acid hydrolysis to yield a hydrolysate (a hyaluronic acid and/or a salt thereof) having a smaller molecular weight.

(Reaction Temperature)

In the liquid phase decomposition step, typically, when the temperature of a reaction liquid is preferably 85°C or less (preferably 80°C or less), more preferably 60°C or more (preferably 65°C or more), browning of the hyaluronic acid and/or the salt thereof having a reduced molecular weight can be prevented.

(Reaction Time)

In the liquid phase decomposition step, typically, when the reaction time is preferably 30 minutes or more and 10 hours or less, more preferably 60 minutes or more and 8 hours or less, browning of the hyaluronic acid and/or the salt thereof having a reduced molecular weight can be prevented.

(Molecular Weight of Hyaluronic Acid and/or Salt to be Obtained)

The liquid phase decomposition step can provide a hyaluronic acid and/or a salt thereof having an average molecular weight of 3,500 or less (having an average molecular weight of preferably 2,800 or less, more preferably 2,500 or less, and also having an average molecular weight of preferably 1,000 or more, more preferably 1,200 or more).

<Anion-Exchange Resin Treatment Step>

In the production method according to the embodiment of the present invention, an acidic solution containing a hydrolysate obtained through the acid hydrolysis is treated with an anion-exchange resin after the liquid phase decomposition step. In this step, the hydrolysate is a hyaluronic acid and/or a salt thereof obtained by reducing the molecular weight of the raw material hyaluronic acid and/or the salt thereof and having a molecular weight smaller than that of the raw material hyaluronic acid and/or the salt thereof.

The anion-exchange resin treatment step can remove anions (for example, halide ions, such as chloride ions, bromide ions, fluoride ions, or iodide ions, and sulfide ions, such as sulfate ions) in the acidic solution, and hence can reduce the contents of elements derived from anions (for example, chlorine and sulfur) contained in a hyaluronic acid and/or a salt thereof to be finally obtained by the production method according to the embodiment of the present invention.

In the anion-exchange resin treatment step, the acidic solution containing the hydrolysate obtained in the liquid phase decomposition step is treated with an anion-exchange resin after the liquid phase decomposition step, and hence the efficiency of the treatment is excellent. In this case, as the anion-exchange resin used, there are given, for example, a strongly basic ion-exchange resin, a weakly basic ion-exchange resin, a gel ion-exchange resin, and a porous ion-exchange resin. To reduce damage to the resin, the acidic solution may be treated with the anion-exchange resin after an alkali is added to the acidic solution to increase the pH of the acidic solution (pH less than 7) .

In addition, in the anion-exchange resin treatment step, for example, the anion-exchange resin may be added to the acidic solution after the retention (acid hydrolysis), followed by stirring. Anions (for example, the anions described above) may be removed by, for example, fixing the anion-exchange resin to a container, adding the acidic solution to the container, and stirring the resultant to bring the hyaluronic acid and/or the salt thereof in the acidic solution into contact with the anion-exchange resin. In this case, the anion-exchange resin may be fixed to a container. When the anion-exchange resin is fixed, the anion-exchange resin can be easily separated from the acidic solution after the treatment. Alternatively, the acidic solution after the retention (acid hydrolysis) may be passed through a column filled with the anion-exchange resin.

The liquid obtained in the liquid phase decomposition step (for example, the anion-exchange resin treatment step) may be subjected to spray-drying or freeze-drying after adjustment of pH. In this case, when the pH of the liquid is preferably adjusted to 4 or more and 8 or less (preferably 5 or more or 7 or less), browning of the hyaluronic acid and/or the salt thereof to be finally obtained can be prevented, and stability can be enhanced.

The pH may be adjusted with a basic substance (for example: a basic inorganic salt including a hydroxide of an alkali metal or an alkaline earth metal, such as sodium hydroxide, potassium hydroxide, magnesium hydroxide, or calcium hydroxide, a carbonate of an alkali metal or an alkaline earth metal, such as sodium carbonate, potassium carbonate, calcium carbonate, or magnesium carbonate, a bicarbonate of an alkali metal or an alkaline earth metal, such as sodium bicarbonate, potassium bicarbonate, calcium bicarbonate, or magnesium bicarbonate, or a basic organic salt).

<Spray-drying Step or Freeze-drying Step>

The production method according to the embodiment of the present invention includes, after the anion-exchange resin treatment step, subjecting the hydrolysate treated with the resin (treated with the anion-exchange resin) to spray-drying or freeze-drying.

<Spray-drying Step>

In the spray-drying step, the liquid containing the hydrolysate is subjected to spray-drying to prepare the hyaluronic acid and/or a salt thereof in a solid state. That is, the spray-drying step can provide a hyaluronic acid and/or a salt thereof having a molecular weight of 3,500 or less, a proportion of disaccharide of 1 mass% or more and 30 mass% or less, a content of chlorine of 1 mass% or less, a content of sulfur of 1 mass% or less, and a bulk density of more than 0.25 $g/cm^3$.

That is, the spray-drying step can provide the hyaluronic acid and/or the salt thereof according to the above-mentioned embodiment. When the liquid containing the hydrolysate and obtained after the liquid phase decomposition step and the anion-exchange resin treatment step is subjected to spray-drying in the spray-drying step, the content of anions in the hyaluronic acid and/or the salt thereof to be finally obtained can be reduced, and bulky particles of hyaluronic acid and/or salt thereof having a small variation in size can be obtained.

(Concentration of Hyaluronic Acid and/or Salt Thereof)

More specifically, in the spray-drying step, when the liquid to be subjected to spray-drying contains preferably 8 mass% or more and 35 mass% or less, more preferably 10 mass% or more, even more preferably 11 mass% or more of the hydrolysate, and also contains preferably 30 mass% or less, more preferably 28 mass% or less, even more preferably 26 mass% or less, particularly preferably 24 mass% or less of the hydrolysate, the spraying can be carried out appropriately.

In this step, the amount of the hydrolysate contained in the liquid to be subjected to spray-drying is a value calculated on the assumption that the content of the raw material hyaluronic acid and/or the salt thereof contained in the acidic raw material hyaluronic acid solution in the liquid phase decomposition step is the content of the hydrolysate.

(Spray-drying Device)

In the spray-drying step, spray-drying may be carried out using a commercially available spray-drying device. As the commercially available spray-drying device, a disc spray dryer or a vane spray dryer is given.

(Spraying Temperature)

In the spray-drying step, typically, when the temperature of a heater in the spray-drying step is preferably 200 °C or less (preferably 170°C or less), more preferably 150°C or more (preferably 100 °C or more), freezing and browning of the hyaluronic acid and/or the salt thereof can be prevented.

A hyaluronic acid and/or a salt thereof having an average molecular weight of 3,500 or more has a high viscosity. Therefore, when the hyaluronic acid and/or the salt thereof is purified by spray-drying, typically, the liquid containing the hyaluronic acid and/or the salt thereof is required to have a reduced viscosity (for example, the concentration of the hyaluronic acid and/or the salt thereof is typically adjusted to 1 mass% or more and 8 mass% or less) in order to perform spraying appropriately.

Meanwhile, the hydrolysate has a low viscosity. Accordingly, a liquid obtained in the anion-exchange resin treatment step can be sprayed without dilution (or after dilution with a small volume of a diluent) in the spray-drying step, and hence high efficiency is provided.

<Freeze-drying Step>

More specifically, in the freeze-drying step, when the liquid to be subjected to freeze-drying contains preferably 8 mass% or more and 35 mass% or less, more preferably 10 mass% or more, even more preferably 11 mass% or more of the hydrolysate, and also contains more preferably 28 mass% or less, even more preferably 26 mass% or less, particularly preferably 24 mass% or less of the hydrolysate, the freezing can be carried out appropriately.

In this step, the amount of the hydrolysate contained in the liquid to be subjected to freeze-drying is a value calculated on the assumption that the content of the raw material hyaluronic acid and/or the salt thereof contained in the acidic raw material hyaluronic acid solution in the liquid phase decomposition step (or the content of the starting raw material hyaluronic acid and/or the salt thereof contained in the acidic raw material hyaluronic acid solution in the liquid phase decomposition step) is the content of the hydrolysate.

(Freeze-drying Device)

In the freeze-drying step, the freeze-drying may be carried out using a commercially available freeze-drying device. As the commercially available freeze-drying device, a freeze-drying machine (DFR-5N-B) manufactured by ULVAC, Inc. is given.

(Freeze-drying Temperature)

In the freeze-drying step, typically, when the temperature in the freeze-drying step is preferably -20°C or less (preferably -30°C or less), more preferably -80°C or more (preferably -70°C or more), browning of the hyaluronic acid and/or the salt thereof can be prevented.

<Solid Phase Decomposition Step>

The production method according to the embodiment of the present invention may further include, before the liquid phase decomposition step (first molecular-weight reduction step), as a second molecular-weight reduction step, dispersing a starting raw material hyaluronic acid and/or a salt thereof having an average molecular weight of 3,000 or more and 500,000 or less in an acidic water-containing medium to yield the raw material hyaluronic acid and/or the salt thereof (hereinafter sometimes referred to as "solid phase decomposition step").

In the solid phase decomposition step, the molecular weight of the starting raw material hyaluronic acid and/or the salt thereof is reduced to yield the raw material hyaluronic acid and/or the salt thereof. More specifically, in the solid phase decomposition step, when the starting raw material hyaluronic acid and/or the salt thereof is dispersed in the acidic water-containing medium, the starting raw material hyaluronic acid and/or the salt thereof in a solid state (more specifically, a state in which the starting raw material hyaluronic acid and/or the salt thereof is suspended in the acidic water-containing medium) can be subjected to a reaction with an acid without dissolving at least a part of the starting raw material hyaluronic acid and/or the salt thereof in the acidic water-containing medium. This can prevent browning of the hyaluronic acid and/or the salt thereof to be obtained and can prevent generation of a by-product (a product obtained by decomposing the hyaluronic acid, such as monosaccharide or a non-reducing end sugar), and hence the purity of the hyaluronic acid and/or the salt thereof to be obtained can be improved.

In addition, as a result of the solid phase decomposition step, at least a part of the starting raw material hyaluronic acid and/or the salt thereof in a solid state can be subjected to a reaction with an acid without being dissolved in the acidic water-containing medium, and hence generation of monosaccharide by a reaction of the hyaluronic acid and/or the salt thereof having a reduced molecular weight and being dissolved in the reaction liquid with the acid can be suppressed. Thus, the proportion of disaccharide in the hydrolysate (a hyaluronic acid and/or a salt thereof) to be finally obtained through the subsequent liquid phase decomposition step can be adjusted to 1 mass% or more and 30 mass% or less, the proportion of octasaccharide in the hydrolysate (a hyaluronic acid and/or a salt thereof) to be finally obtained through the subsequent liquid phase decomposition step can be adjusted to 1 mass% or more and 30 mass% or less, and the total proportion of disaccharide, tetrasaccharide, hexasaccharide, and octasaccharide in the hydrolysate (a hyaluronic acid and/or a salt thereof) tobefinally obtained through the subsequent liquid phase decomposition step can be adjusted to 5 mass% or more and 80 mass% or less.

When a hyaluronic acid and/or a salt thereof having an average molecular weight of 3,500 or less is produced by dispersing a starting raw material hyaluronic acid and/or a salt thereof having an average molecular weight of 10,000 or more and 500,000 or less in an acidic water-containing medium, a hyaluronic acid and/or a salt thereof having a reduced molecular weight, due to its higher solubility, preferentially dissolves in water that constitutes the acidic water-containing medium, and as a result, browning of a hyaluronic acid and/or a salt thereof to be finally obtained may be caused or the amount of a by-product (for example, monosaccharide) generated may increase (for example, the content of the monosaccharide may exceed 3 mass%).

Meanwhile, in the production method according to the embodiment of the present invention, browning and generation of a by-product can be suppressed by reducing the molecular weight of the starting raw material hyaluronic acid and/or the salt thereof to some extent in the solid phase decomposition step and then subjecting the resultant to the liquid phase decomposition step to yield a hyaluronic acid and/or a salt thereof having an average molecular weight of 3,500 or less.

(Starting Raw Material Hyaluronic Acid and/or Salt Thereof)

The starting raw material hyaluronic acid and/or the salt thereof is a starting raw material of the raw material hyaluronic acid and/or the salt thereof and has an average molecular weight larger than that of the raw material hyaluronic acid and/or the salt thereof.

When the average molecular weight of the starting raw material hyaluronic acid and/or the salt thereof is preferably 10,000 or more, more preferably 100,000 or more and is also preferably 2,000,000 or less, more preferably 1,000,000 or less, the raw material hyaluronic acid and/or the salt thereof having an average molecular weight of 3,500 or more and 500,000 or less can be obtained.

In the solid phase decomposition step, the mass of the starting raw material hyaluronic acid and/or the salt thereof with respect to the mass of the acidic water-containing medium (the mass of the acidic water-containing medium is defined as 100 mass%) may be 3 mass% or more and 20 mass% or less, and is preferably 3 mass% or more and is also preferably 15 mass% or less.

(Acidic Water-containing Medium)

In the production method according to the embodiment of the present invention, as a solvent to be used in the acidic water-containing medium in which the starting raw material hyaluronic acid and/or the salt thereof is dispersed, a water-soluble organic solvent is given. Examples of the water-soluble organic solvent may include: alcohol-based solvents, such as methanol, ethanol, 1-propanol, and 2-propanol (of those, a lower alcohol) ; ketone-based solvents, such as acetone and methyl ethyl ketone; tetrahydrofuran; and acetonitrile. Those solvents may be used alone or in a combination.

(Proportion of Water)

When the proportion of water with respect to the total volume of the acidic water-containing medium is preferably 40 vol% or less, more preferably 30 vol% or less and is typically 1 vol% or more, dissolution of the raw material hyaluronic acid and/or the salt thereof in the acidic water-containing medium is prevented and a decrease in the yield is avoided.

(pH of Acidic Water-containing Medium)

In addition, in the solid phase decomposition step, when the pH of the acidic water-containing medium is preferably 1 or less, more preferably 0 or more, browning of the raw material hyaluronic acid and/or the salt thereof can be prevented, and the molecular weight of the raw material hyaluronic acid and/or the salt thereof can be reduced.

(Second Molecular-weight Reduction Step except Solid Phase Decomposition Step)

The molecular weight of the starting raw material hyaluronic acid and/or the salt thereof may be reduced by using, in the second molecular-weight reduction step instead of the solid phase decomposition step, for example, at least one kind of method selected from enzymatic decomposition, thermal decomposition, acid decomposition under a state in which the raw material hyaluronic acid and/or the salt thereof is dissolved in a reaction liquid, alkaline decomposition, decomposition by electron beam irradiation, and ultrasonic decomposition, to thereby prepare the raw material hyaluronic acid and/or the salt thereof. In particular, when the solid phase decomposition step (that is, the starting raw material hyaluronic acid and/or the salt thereof is dispersed in the acidic water-containing medium, and the starting raw material hyaluronic acid and/or the salt thereof in a solid state is subjected to a reaction with an acid) is preferable in order to hydrolyze the starting raw material hyaluronic acid and/or the salt thereof, the proportions of monosaccharide, disaccharide, and octasaccharide in the hyaluronic acid and/or the salt thereof to be finally obtained can be easily controlled.

(Reaction Temperature)

In the solid phase decomposition step, typically, when the temperature of the reaction liquid is preferably 95°C or less (preferably 90°C or less), more preferably 55°C or more (preferably 60°C or more), browning of the hyaluronic acid and/or the salt thereof having a reduced molecular weight can be prevented.

(Reaction Time)

In the solid phase decomposition step, typically, when the reaction time is preferably 30 minutes or more and 10 hours or less, more preferably 60 minutes or more and 2 hours or less, browning of the hyaluronic acid and/or the salt thereof having a reduced molecular weight can be prevented.

<Action and Effect>

The production method according to the embodiment of the present invention includes the liquid phase decomposition step, the anion-exchange resin treatment step, and the spray-drying step or the freeze-drying step. Accordingly, the liquid obtained in the anion-exchange resin treatment step can be subjected to the spray-drying step or the freeze-drying step, and hence the hyaluronic acid and/or the salt thereof according to the above-mentioned embodiment can be efficiently obtained as a solid.

More specifically, according to the production method of the embodiment of the present invention, a hyaluronic acid and/or a salt thereof having a high bulk density can be obtained by subjecting a liquid containing a hyaluronic acid and/or a salt thereof having an average molecular weight of 3,500 or less at a high concentration (for example, 8 mass% or more and 35 mass% or less) to spray-drying or freeze-drying.

In the production method according to the embodiment of the present invention, when a hyaluronic acid and/or a salt thereof having an average molecular weight of 3,500 or less, a high bulk density (0.25 g/cm$^3$ or more), and a small variation in particle diameter can be obtained through the spray-drying step, the hyaluronic acid and/or the salt thereof having an average molecular weight of 3,500 or less has a low viscosity, and hence the concentration of the hyaluronic acid and/or the salt thereof can increase before spray-drying. Accordingly, the spraying can be appropriately carried out.

In order to prepare a solid by the spray-drying, typically, it is necessary to use a liquid containing an appropriate concentration of a substance to be subjected to the spray-drying and having a low viscosity in the spray-drying. When the concentration of the substance to be subjected to the spray-drying is too low, the substance to be subjected to the spray-drying may be hardly obtained as a solid.

A hyaluronic acid and/or a salt thereof having a high molecular weight (for example, an average molecular weight of more than 3,500, in particular, an average molecular weight of 100,000 or more and 2,000,000 or less) has a high viscosity, and hence, in order to perform the spray-drying, it is necessary to reduce the concentration of the hyaluronic acid and/or the salt thereof to decrease the viscosity. However, when such liquid containing a low concentration of the hyaluronic acid and/or the salt thereof is subjected to the spray-drying, a problem arises in that the hyaluronic acid may not be collected as a solid.

Meanwhile, the inventors of the present invention have discovered novel findings that spray-drying can be carried out by adjusting the concentration of a hyaluronic acid and/or a salt thereof having an average molecular weight of 3,500 or less in a liquid to be subjected to the spray-drying to 8 mass% or more and 35 mass% or less, and have created the production method according to the embodiment of the present invention.

That is, the hyaluronic acid and/or the salt thereof according to the above-mentioned embodiment can be obtained by the production method according to the embodiment of the present invention.

In addition, the production method according to the embodiment of the present invention has the following characteristics (1) to (5).

(1) Compared to a method of reducing the molecular weight of a hyaluronic acid and/or a salt thereof with an enzyme, according to the production method of the embodiment of the present invention, it is possible to produce a hyaluronic acid and/or a salt thereof having a high purity and a high degree of whiteness, to reduce antigenicity, and to reduce a production cost.

(2) Compared to a method of purifying a hyaluronic acid and/or a salt thereof by precipitation through addition of ethanol, according to the production method of the embodiment of the present invention, a hyaluronic acid and/or a salt thereof having a low content of trace impurities (for example, a chloride ion) can be obtained, and the yield of the hyaluronic acid and/or the salt thereof is excellent.

(3) Compared to a method of producing a hyaluronic acid and/or a salt thereof having an average molecular weight of 3,500 or less by reducing the molecular weight of a raw material hyaluronic acid and/or a salt thereof having an average molecular weight of more than 100,000 under a state in which the hyaluronic acid and/or the salt thereof is dissolved in an acidic or alkaline raw material hyaluronic acid solution, according to the production method of the embodiment of the present invention, a hyaluronic acid and/or a salt thereof having a high degree of whiteness and a low content of by-products (for example, monosaccharide) can be obtained.

(4) When a hyaluronic acid and/or a salt thereof having an average molecular weight of 3,500 or less is produced by a method involving dispersing, in a solid state, a raw material hyaluronic acid and/or a salt thereof having an average molecular weight of more than 100,000 in an acidic water-containing medium (as the method involving dispersing, in a solid state, a raw material hyaluronic acid and/or a salt thereof in an acidic water-containing medium, for example, the method described as the solid phase decomposition step is given), the resultant low-molecular-weight product (a hyaluronic acid and/or a salt thereof having an average molecular weight of 3,500 or less) dissolves in acidic water that constitutes the acidic water-containing medium depending on the conditions, and hence the recovery is difficult, browning of the low-molecular-weight product is caused due to an acid contained in the acidic water, or a by-product, such as monosaccharide, is generated by decomposition of the low-molecular-weight product with the acid in some cases. On the other hand, according to the production method of the embodiment of the present invention, a hyaluronic acid and/or a salt thereof having a high degree of whiteness and a low content of a by-product (for example, monosaccharide) can be obtained.

(5) In particular, the hyaluronic acid and/or the salt thereof obtained through the spray-drying step has a smaller variation in particle diameter and a lower content of trace impurities compared to a hyaluronic acid and/or a salt thereof obtained through the freeze-drying step (typically, a fluffy cotton-like solid is obtained).

[Spray-drying/Freeze-drying of Low-molecular-weight Hyaluronic Acid]

A method of producing a hyaluronic acid and/or a salt thereof according to one embodiment of the present invention includes subjecting a liquid containing 8 mass% or more and 35 mass% or less of a hyaluronic acid and/or a salt thereof having an average molecular weight of 3,500 or less to spray-drying or freeze-drying to obtain a hyaluronic acid and/or a salt thereof in a solid state.

In the production method according to the embodiment of the present invention, when a hyaluronic acid and/or a salt thereof in a solid state is preferably obtained by spray-drying, particles having a higher bulk density and having a small variation in particle diameter can be obtained. In this case, the average molecular weight of the hyaluronic acid and/or the salt thereof to be subjected to spray-drying is preferably 3,500 or less, more preferably 2,800 or less, and is even more preferably 1,000 or more, more preferably 1,200 or more.

That is, the hyaluronic acid and/or the salt thereof having an average molecular weight of 3,500 or less has a low viscosity compared to a high-molecular-weight hyaluronic acid and/or a salt thereof. Accordingly, when the concentration of the hyaluronic acid and/or the salt thereof is increased (adjusted to 8 mass% or more and 35 mass% or less) in the liquid to be subjected to the spray-drying in the production method according to the embodiment of the present invention, a particulate hyaluronic acid and/or a salt thereof having a high bulk density and a small variation in particle diameter can be obtained.

[One Example of Hyaluronic Acid and/or Salt Thereof]

A hyaluronic acid and/or a salt thereof obtained by the method of the present invention has an average molecular weight of 3,500 or less and has one or more (two, three, four, five, six, seven, eight, nine, ten, eleven, twelve, or thirteen) of the following characteristics (1) to (13):

(1) a bulk density of more than 0.25 g/cm$^3$;
(2) a purity of 90% or more;
(3) a b value indicating the hue of a color of 10 or less;
(4) an average particle diameter of 10 μm or more and 35 μm or less;
(5) a standard deviation of particle diameters of 0.1 or more and 0.3 or less;
(6) a content of chlorine of 1 mass% or less;
(7) a content of sulfur of 1 mass% or less;
(8) a proportion of disaccharide of 1 mass% or more and 30 mass% or less;
(9) no hyaluronic acid-degrading enzyme content;
(10) a proportion of octasaccharide of 1 mass% or more and 30 mass% or less;
(11) a total proportion of disaccharide, tetrasaccharide, hexasaccharide, and octasaccharide of 5 mass% or more and 80 mass% or less;
(12) a kinetic viscosity in a 5 mass% aqueous solution of 2.0 mm$^2$/s or less; and
(13) a proportion of monosaccharide of 3 mass% or less.

The hyaluronic acid and/or the salt thereof according to the embodiment of the present invention has the same action and effect as those of the hyaluronic acid and/or the salt thereof according to the above-mentioned embodiment.

[Examples]

The present invention is hereinafter described in more detail by way of Examples. However, the present invention is by no means limited to Examples.

<Example 1: Preparation of Hyaluronic Acid>

About 5 kg of a raw material hyaluronic acid having a molecular weight of 10,000 was moistened with 1 kg of ethanol, and the resultant was added to 25 L of 2% hydrochloric acid (pH 1.0, 25 kg) in a 50 L tank with a stirrer and a jacket to prepare an acidic aqueous solution of the raw material hyaluronic acid (pH about 1) containing about 16 mass% of the raw material hyaluronic acid. The resultant was retained at 70°C for 5 hours to be subjected to hydrolysis (liquid phase decomposition step) . In addition, the resultant liquid containing a hydrolysate was treated with an anion-exchange resin (trade name: DIAION or SEPABEADS, manufactured by Mitsubishi Chemical Corporation), and was pushed out of the column and diluted with 8 L of water, followed by removal of the resin using an 80 mesh filter. 2 kg of a 25% aqueous solution of sodium hydroxide was added thereto to adjust the pH to 4.0 or more and 6.5 or less. The resultant solution (the concentration of the hyaluronic acid: 12 mass%, in this example, the concentration of the hyaluronic acid was calculated on the assumption that the amount of the raw material hyaluronic acid added was the content of the hyaluronic acid) was dried by spray-drying (temperature of a heater: 180°C). Thus, 4.5 kg (yield: about 90%) of a hyaluronic acid was obtained as fine white powder.

The resultant hyaluronic acid had a molecular weight of 2,200, as determined by conversion through HPLC measurement, a b value of 2.2, and an L value of 95. In addition, the bulk density, the particle diameter, the particle size distribution, and the content of chlorine of the hyaluronic acid were measured, and the results are shown in Table 1.

In addition, the hyaluronic acid of Example 1 and the hyaluronic acids of Examples 2 to 8 and Comparative Examples 2 to 4, 7, and 8 to be described later were subjected to HPLC measurement. The conditions of the HPLC measurement are as described below. The average molecular weights and the proportions of disaccharide, tetrasaccharide, hexasaccharide, and octasaccharide of the hyaluronic acids of Examples 1 to 8 and the hyaluronic acids of Comparative Examples 2 to 4, 7, and 8 to be described later were calculated from the results of the HPLC measurement in accordance with the method described in the section of the above-mentioned embodiment.

(HPLC Measurement Conditions)

HPLC analyzing apparatus: trade name "Alliance PDA System", manufacturing by Nihon Waters K.K.
Used column: gel filtration column (trade name "Diol-120", manufacturing by YMC Co., Ltd).
Analyzed sample: a 0.1% (w/v) aqueous solution of hyaluronic acid
Column temperature: 40°C
Flow rate: 1 mL/min

Injected volume of a 0.1% (w/v) aqueous solution of hyaluronic acid: 20 μL
Mobile phase: 0.003 M phosphate buffer (containing 0.15 M NaCl, pH 7.0)

In liquid chromatography using a gel filtration column according to this example, a sample having a longer retention time has a lower molecular weight. Accordingly, in the results of HPLC measurement for the hyaluronic acids, peaks of N-acetylglucosamine, D-glucuronic acid, a hyaluronic acid (disaccharide: one repeating structural unit), a hyaluronic acid (tetrasaccharide: two repeating structural units), a hyaluronic acid (hexasaccharide: three repeating structural units), a hyaluronic acid (octasaccharide: four repeating structural units), and ··· appeared in descending order of retention time. The molecular weights of the peaks (for example, in the case of disaccharide, molecular weight: 400) are multiplied by peak areas, and the values, which are calculated for the respective peaks, are integrated. Further, the resultant integrated value may be divided by the total of the peak areas. Thus, an average molecular weight may be calculated.

<Comparative Example 1: Preparation of Hyaluronic Acid>

About 2 kg of a dried raw material hyaluronic acid having a molecular weight of 10,000 was added to 25 L of 2% hydrochloric acid (pH 1.0, 25 kg) in a 50 L tank with a stirrer and a jacket to prepare an acidic aqueous solution of the raw material hyaluronic acid (pH about 1) containing about 7 mass% of the raw material hyaluronic acid. The resultant was retained at 70°C for 5 hours to be subjected to hydrolysis (liquid phase decomposition step) . Further, 2 kg of a 25 mass% aqueous solution of sodium hydroxide was added to the resultant hydrolysate to adjust the pH to 4.0 or more and 6.5 or less, the resultant was treated with an anion-exchange resin (trade name: DIAION or SEPABEADS, manufactured by Mitsubishi Chemical Corporation), and was pushed out of the column and diluted with 18 L of water, followed by removal of the resin using an 80 mesh filter. When the resultant solution (the concentration of the hyaluronic acid: 4 mass%, in this comparative example, the concentration of the hyaluronic acid was calculated on the assumption that the amount of the raw material hyaluronic acid added was the content of the hyaluronic acid) was dried by spray-drying (temperature of a heater: 180°C), the solution was turned into a fine mist, and hence the hyaluronic acid could not be collected.

<Comparative Example 2: Preparation of Hyaluronic Acid>

1.9 kg of a hyaluronic acid of Comparative Example 2 was obtained as fine white powder in the same manner as in Comparative Example 1 except that freeze-drying was carried out instead of the spray-drying.

<Example 2: Preparation of Hyaluronic Acid>

A 200 L tank with a stirrer and a jacket was filled with 28 L (28 kg) of 8% hydrochloric acid and 100 L of ethanol, and the liquid was heated to a temperature of 70°C while being stirred. After the temperature reached 70°C, 5 kg of a starting raw material hyaluronic acid having an average molecular weight of 300,000 was loaded into the tank while the liquid was stirred. The liquid was stirred for 1 hour so that the hyaluronic acid fine powder was in a dispersion state while the liquid was heated so that the temperature of the aqueous ethanol containing hydrochloric acid (pH 0.2) was maintained at 70°C (solid phase decomposition step). Subsequently, the aqueous ethanol containing hydrochloric acid was removed by decantation, and 25 L of 2% hydrochloric acid (pH 1.0, 25 kg) was added to about 6 kg of the residual solid matter (the raw material hyaluronic acid moistened with 1 kg of ethanol) to prepare an acidic aqueous solution of the raw material hyaluronic acid (pH about 1) containing about 16 mass% of the raw material hyaluronic acid, which was subjected to hydrolysis at 70°C for 5 hours (liquid phase decomposition step). Further, the resultant liquid containing a hydrolysate was treated with an anion-exchange resin (trade name: DIAION or SEPABEADS, manufactured by Mitsubishi Chemical Corporation), and was pushed out of the column and diluted with 12 L of water, followed by removal of the resin using an 80 mesh filter. 2 kg of a 25% aqueous solution of sodium hydroxide was added thereto to adjust the pH to 4.0 or more and 6.5 or less. The resultant solution (the concentration of the hyaluronic acid: 11 mass%, in this example, the concentration of the hyaluronic acid was calculated on the assumption that the amount of the starting raw material hyaluronic acid added was the content of the hyaluronic acid) was dried by spray-drying (temperature of a heater: 180°C). Thus, 4.5 kg (yield: about 90%) of a hyaluronic acid was obtained as fine white powder. The average molecular weight of the raw material hyaluronic acid obtained in the solid phase decomposition step was separately measured and found to be about 10,000.

<Example 3: Preparation of Hyaluronic Acid>

A hyaluronic acid of Example 3 was obtained in the same manner as in Example 2 except that, in Example 2, each of the volume of the tank used, the mass of the starting raw material hyaluronic acid, the mass of hydrochloric acid, the mass of ethanol, and the mass of sodium hydroxide, were scaled down to one-tenth, and the concentration of the hyaluronic acid solution used in the spray-drying was changed to 14 mass%.

More specifically, a 2 L beaker was filled with 280 mL (280 g) of 8% hydrochloric acid and 1 L of ethanol, and the liquid was heated to a temperature of 70°C in a hot-water bath while being stirred. After the temperature reached 70°C, 50 g of a starting raw material hyaluronic acid having an average molecular weight of 300,000 was loaded into the beaker while the liquid was stirred. The liquid was stirred for 1 hour so that the hyaluronic acid fine powder was in a dispersion state while the liquid was heated so that the temperature of the aqueous ethanol containing hydrochloric acid (pH0.2) was maintained at 70°C (solid phase decomposition step). Subsequently, the aqueous ethanol containing hydrochloric acid was removed by decantation, and 150 mL of 2% hydrochloric acid (pH 1.0, 150 g) was added to about 60 g of the residual solid matter (the raw material hyaluronic acid moistened with 10 g of ethanol) to prepare an acidic aqueous solution of the raw material hyaluronic acid containing about 24 mass% of the raw material hyaluronic acid (pH about 1), which was subjected to hydrolysis at 70°C for 5 hours (liquid phase decomposition step). Further, the resultant liquid containing a hydrolysate was treated with an anion-exchange resin (trade name: DIAION or SEPABEADS, manufactured by Mitsubishi Chemical Corporation), and was pushed out of the column and diluted with 120 mL of water, followed by removal of the resin using an 80 mesh filter. 20 g of a 25% aqueous solution of sodium hydroxide was added thereto to adjust the pH to 4.0 or more and 6.5 or less. The resultant solution (the concentration of the hyaluronic acid: 14 mass%, in this example, the concentration of the hyaluronic acid was calculated on the assumption that the amount of the starting raw material hyaluronic acid added was the content of the hyaluronic acid) was dried by spray-drying (temperature of a heater: 180°C). Thus, 46 g (yield: about 92%) of a hyaluronic acid was obtained as fine white powder. The average molecular weight of the raw material hyaluronic acid obtained in the solid phase decomposition step was separately measured and found to be about 10,000.

<Example 4: Preparation of Hyaluronic Acid>

A solution containing 11 mass% of a hyaluronic acid of Example 4 was obtained in the same manner as in Example 3 except that, in Example 3, the concentration of the hyaluronic acid was adjusted by adding water to the solution obtained after removal of the resin. The solution was subjected to freeze-drying to yield a hyaluronic acid of Example 4.

More specifically, a 2 L beaker was filled with 280 mL (280 g) of 8% hydrochloric acid and 1 L of ethanol, and the liquid was heated to a temperature of 70°C in a hot-water bath while being stirred. After the temperature reached 70°C, 50 g of a starting raw material hyaluronic acid having an average molecular weight of 300,000 was loaded into the beaker while the liquid was stirred. The liquid was stirred for 1 hour so that the hyaluronic acid fine powder was in a dispersion state while the liquid was heated so that the temperature of the aqueous ethanol containing hydrochloric acid (pH0.2) was maintained at 70°C (solid phase decomposition step). Subsequently, the aqueous ethanol containing hydrochloric acid was removed by decantation, and 150 mL of 2% hydrochloric acid (pH 1.0, 150 g) was added to about 60 g of the residual solid matter (the raw material hyaluronic acid moistened with 10 g of ethanol) to prepare an acidic aqueous solution of the raw material hyaluronic acid containing about 24 mass% of the raw material hyaluronic acid (pH about 1), which was subjected to hydrolysis at 70°C for 5 hours (liquid phase decomposition step). Further, the resultant liquid containing a hydrolysate was treated with an anion-exchange resin (trade name: DIAION or SEPABEADS, manufactured by Mitsubishi Chemical Corporation), and was pushed out of the column and diluted with 120 mL of water, followed by removal of the resin using an 80 mesh filter. 20 g of a 25% aqueous solution of sodium hydroxide was added thereto to adjust the pH to 4.0 or more and 6.5 or less. A solution obtained by diluting a part of the resultant solution 1.25 times (the concentration of the hyaluronic acid: 11 mass%, in this example, the concentration of the hyaluronic acid was calculated on the assumption that the amount of the starting raw material hyaluronic acid added was the content of the hyaluronic acid) was dried by freeze-drying. Thus, a hyaluronic acid was obtained as fine white powder. The average molecular weight of the raw material hyaluronic acid obtained in the solid phase decomposition step was separately measured and found to be about 10,000.

<Comparative Example 3: Preparation of Hyaluronic Acid>

A solution containing about 1 mass% (0.6 mass%) of a hyaluronic acid of Comparative Example 3 was obtained in the same manner as in Example 2 mainly, except that, in Example 2, the concentration of the hyaluronic acid was adjusted by adding water to the solution obtained after removal of the resin. The solution was subjected to freeze-drying to yield a hyaluronic acid of Comparative Example 3.

More specifically, a 2 L beaker was filled with 280 mL (280 g) of 8% hydrochloric acid and 1 L of ethanol, and the liquid was heated to a temperature of 70°C in a hot-water bath while being stirred. After the temperature reached 70°C, 50 g of a starting raw material hyaluronic acid having an average molecular weight of 300,000 was loaded into the beaker while the liquid was stirred. The liquid was stirred for 1 hour so that the hyaluronic acid fine powder was in a dispersion state while the liquid was heated so that the temperature of the aqueous ethanol containing hydrochloric acid (pH0.2) was maintained at 70°C (solid phase decomposition step). Subsequently, the aqueous ethanol containing hydrochloric acid was removed by decantation, and 150 mL of 2% hydrochloric acid (pH 1.0, 150 g) was added to about 60 g of the residual solid matter (the raw material hyaluronic acid moistened with 10 g of ethanol) to prepare an acidic aqueous solution of the raw material hyaluronic acid containing about 24 mass% of the raw material hyaluronic acid (pH about 1), which was subjected to hydrolysis at 70°C for 5 hours (liquid phase decomposition step). Further, the resultant liquid containing a hydrolysate was treated with an anion-exchange resin (trade name: DIAION or SEPABEADS, manufactured by Mitsubishi Chemical Corporation), and was pushed out of the column and diluted with 120 mL of water, followed by removal of the resin using an 80 mesh filter. 20 g of a 25% aqueous solution of sodium hydroxide was added thereto to adjust the pH to 4.0 or more and 6.5 or less. A solution obtained by diluting a part of the resultant solution 22 times (the concentration of the hyaluronic acid: 0.6 mass%, in this comparative example, the concentration of the hyaluronic acid was calculated on the assumption that the amount of the starting raw material hyaluronic acid added was the content of the hyaluronic acid) was dried by freeze-drying. Thus, a hyaluronic acid was obtained as fine white powder. The average molecular weight of the raw material hyaluronic acid obtained in the solid phase decomposition step was separately measured and found to be about 10,000.

<Comparative Example 4: Preparation of Hyaluronic Acid>

A solution containing 6 mass% of a hyaluronic acid of Comparative Example 4 was obtained in the same manner as in Comparative Example 3 except that, in Comparative Example 3, the amount of water added to the solution obtained after removal of the resin was changed. The solution was subjected to freeze-drying to yield a hyaluronic acid of Comparative Example 4.

More specifically, a 2 L beaker was filled with 280 mL (280 g) of 8% hydrochloric acid and 1 L of ethanol, and the liquid was heated to a temperature of 70°C in a hot-water bath while being stirred. After the temperature reached 70°C, 50 g of a starting raw material hyaluronic acid having an average molecular weight of 300,000 was loaded into the beaker while the liquid was stirred. The liquid was stirred for 1 hour so that the hyaluronic acid fine powder was in a dispersion state while the liquid was heated so that the temperature of the aqueous ethanol containing hydrochloric acid (pH0.2) was maintained at 70°C (solid phase decomposition step). Subsequently, the aqueous ethanol containing hydrochloric acid was removed by decantation, and 150 mL of 2% hydrochloric acid (pH 1.0, 150 g) was added to about 60 g of the residual solid matter (the raw material hyaluronic acid moistened with 10 g of ethanol) to prepare an acidic aqueous solution of the raw material hyaluronic acid containing about 24 mass% of the raw material hyaluronic acid (pH about 1), which was subjected to hydrolysis at 70°C for 5 hours (liquid phase decomposition step). Further, the resultant liquid containing a hydrolysate was treated with an anion-exchange resin (trade name: DIAION or SEPABEADS, manufactured by Mitsubishi Chemical Corporation), and was pushed out of the column and diluted with 120 mL of water, followed by removal of the resin using an 80 mesh filter. 20 g of a 25% aqueous solution of sodium hydroxide was added thereto to adjust the pH to 4.0 or more and 6.5 or less. A solution obtained by diluting a part of the resultant solution 2.5 times (the concentration of the hyaluronic acid: 6 mass%, in this comparative example, the concentration of the hyaluronic acid was calculated on the assumption that the amount of the starting raw material hyaluronic acid added was the content of the hyaluronic acid) was dried by freeze-drying. Thus, a hyaluronic acid was obtained as fine white powder. The average molecular weight of the raw material hyaluronic acid obtained in the solid phase decomposition step was separately measured and found to be about 10,000.

<Comparative Example 5: Preparation of Hyaluronic Acid>

A solution containing 6 mass% of a hyaluronic acid of Comparative Example 5 was obtained in the same manner as in Example 2 except that, in Example 2, the amount of water added to the solution obtained after removal of the resin was changed. When the solution was subjected to spray-drying in the same manner as in Example 2, the solution was turned into a fine mist, and hence the hyaluronic acid could not be collected.

More specifically, a 2 L beaker was filled with 280 mL (280 g) of 8% hydrochloric acid and 1 L of ethanol, and the liquid was heated to a temperature of 70°C in a hot-water bath while being stirred. After the temperature reached 70°C, 50 g of a starting raw material hyaluronic acid having an average molecular weight of 300,000 was loaded into the beaker while the liquid was stirred. The liquid was stirred for 1 hour so that the hyaluronic acid fine powder was in a dispersion state while the liquid was heated so that the temperature of the aqueous ethanol containing hydrochloric acid (pH0.2) was maintained at 70°C (solid phase decomposition step). Subsequently, the aqueous ethanol containing hydrochloric acid was removed by decantation, and 150 mL of 2% hydrochloric acid (pH 1.0, 150 g) was added to about 60 g of the residual solid matter (the raw material hyaluronic acid moistened with 10 g of ethanol) to prepare an acidic aqueous solution of the raw material hyaluronic acid containing about 24 mass% of the raw material hyaluronic acid (pH about 1), which was subjected to hydrolysis at 70°C for 5 hours (liquid phase decomposition step). Further, the resultant liquid containing a hydrolysate was treated with an anion-exchange resin (trade name: DIAION or SEPABEADS, manufactured by Mitsubishi Chemical Corporation), and was pushed out of the column and diluted with 120 mL of water, followed by removal of the resin using an 80 mesh filter. 20 g of a 25% aqueous solution of sodium hydroxide was added thereto to adjust the pH to 4.0 or more and 6.5 or less. When a solution obtained by diluting a part of the resultant solution 2.5 times (the concentration of the hyaluronic acid: 6 mass%, in this comparative example, the concentration of the hyaluronic acid was calculated on the assumption that the amount of the starting raw material hyaluronic acid added was the content of the hyaluronic acid) was subjected to spray-drying, the solution was turned into a fine mist, and hence the hyaluronic acid could not be collected.

<Comparative Example 6: Preparation of Hyaluronic Acid>

A 2 L beaker was filled with 280 mL (280 g) of 8% hydrochloric acid and 1 L of ethanol, and the liquid was heated to a temperature of 70°C in a hot-water bath while being stirred. After the temperature reached 70°C, 50 g of a starting raw material hyaluronic acid having an average molecular weight of 300,000 was loaded into the beaker while the liquid was stirred. The liquid was stirred for 1 hour so that the hyaluronic acid fine powder was in a dispersion state while the liquid was heated so that the temperature of the aqueous ethanol containing hydrochloric acid (pH 0.2) was maintained at 70°C (solid phase decomposition step). Subsequently, the aqueous ethanol containing hydrochloric acid was removed by decantation, and the residual solid matter was dried under reduced pressure. When 150 mL of 2% hydrochloric acid (pH 1.0, 150 g) was added to about 50 g of the dried hyaluronic acid, the hyaluronic acid formed aggregates and was not dissolved completely. However, the hyaluronic acid was used without additional treatment to prepare an acidic aqueous solution of the raw material hyaluronic acid (pH about 1) containing about 25 mass% of the raw material hyaluronic acid, which was subjected to hydrolysis at 70°C for 5 hours (liquid phase decomposition step). Further, the resultant liquid containing a hydrolysate was treated with an anion-exchange resin (trade name: DIAION or SEPABEADS, manufactured by Mitsubishi Chemical Corporation), and was pushed out of the column and diluted with 120 mL of water, followed by removal of the resin using an 80 mesh filter. 20 g of a 25% aqueous solution of sodium hydroxide was added thereto to adjust the pH to 4.0 or more and 6.5 or less. When the resultant solution (the concentration of the hyaluronic acid: 15 mass%, in this comparative example, the concentration of the hyaluronic acid was calculated on the assumption that the amount of the starting raw material hyaluronic acid added was the content of the hyaluronic acid) was dried by spray-drying, a brown hyaluronic acid was obtained. The resultant hyaluronic acid had a b value of 10 or more and a proportion of monosaccharide of 4%.

<Comparative Example 7: Preparation of Hyaluronic Acid>

A hyaluronic acid of Comparative Example 7 was obtained in the same manner as in Example 2 except that the time for hydrolysis in the solid phase decomposition step changed to 2 hours, sodium hydroxide was added to neutralize the solution before the treatment with the anion-exchange resin, and ethanol was added to precipitate a hyaluronic acid in Example 2.
More specifically, a 500 L tank with a stirrer and a jacket was filled with 28 L (28 kg) of 8% hydrochloric acid and 100 L of ethanol, and the liquid was heated to a temperature of 70°C while being stirred. After the temperature reached 70°C, 5 kg of a starting raw material hyaluronic acid having an average molecular weight of 300,000 was loaded into the tank while the liquid was stirred. The liquid was stirred for 1 hour so that the hyaluronic acid fine powder was in a dispersion state while the liquid was heated so that the temperature of the aqueous ethanol containing hydrochloric acid (pH0.2) was maintained at 70°C (solid phase decomposition step). Subsequently, the aqueous ethanol containing hydrochloric acid was removed by decantation, and 25 L of 2% hydrochloric acid (pH 1.0, 25 kg) was added to about 6 kg of the residual solid matter (the raw material hyaluronic acid moistened with 1 kg of ethanol) to prepare an acidic aqueous solution of the raw material hyaluronic acid (pH about 1) containing about 16 mass% of the raw material hyaluronic acid, which was subjected to hydrolysis at 70°C for 2 hours (liquid phase decomposition step). 240 L of ethanol was added thereto to suspend the hyaluronic acid, and the pH of the suspension was adjusted to 4.0 or more and 6.5 or less with a 25% solution of sodium hydroxide. Thus, the hyaluronic acid was precipitated. The resultant precipitates were dried under reduced pressure to yield 4.5 kg (yield: about 90%) of a hyaluronic acid as fine white powder. The average molecular weight of the raw material hyaluronic acid obtained in the solid phase decomposition step was separately measured and found to be about 10,000.
It is considered that the hyaluronic acid of Comparative Example 7 was obtained without the anion-exchange resin treatment step, and hence the content of chlorine was more than 1 mass%, and additionally, the hyaluronic acid was obtained by precipitation, and hence the standard deviation of particle diameters was far greater than 0.3, indicating that the shapes of the particles were not uniform.

<Comparative Example 8: Preparation of Hyaluronic Acid>

A 200 L tank with a stirrer and a jacket was filled with 28 L (28 kg) of 8% hydrochloric acid and 100 L of ethanol, and the liquid was heated to a temperature of 70°C while being stirred. After the temperature reached 70°C, 5 kg of a starting raw material hyaluronic acid having an average molecular weight of 300,000 was loaded into the tank while the liquid was stirred. The liquid was stirred for 1 hour so that the hyaluronic acid fine powder was in a dispersion state while the liquid was heated so that the temperature of the aqueous ethanol containing hydrochloric acid (pH 0.2) was maintained at 70°C (solid phase decomposition step). Subsequently, the aqueous ethanol containing hydrochloric acid was removed by decantation, and 25 L of 2% hydrochloric acid (pH 1.0, 25 kg) was added to about 6 kg of the residual solid matter (the raw material hyaluronic acid moistened with 1 kg of ethanol) to prepare an acidic aqueous solution of the raw material hyaluronic acid (pH about 1) containing about 16 mass% of the raw material hyaluronic acid, which was subjected to hydrolysis at 70°C for 5 hours (liquid phase decomposition step). 2 kg of a 25% aqueous solution of sodium hydroxide was added thereto to adjust the pH to 4.0 or more and 6.5 or less. The resultant solution (the concentration of the hyaluronic acid: 15 mass%, in this comparative example, the concentration of the hyaluronic acid was calculated on the assumption that the amount of the starting raw material hyaluronic acid added was the content of the hyaluronic acid) was dried by spray-drying. Thus, 4.5 kg (yield: about 90%) of a hyaluronic acid was obtained as fine white powder. The average molecular weight of the raw material hyaluronic acid obtained in the solid phase decomposition step was separately measured and found to be about 10,000.

It is considered that the hyaluronic acid of Comparative Example 8 was obtained without the anion-exchange resin treatment step, and hence the content of chlorine was more than 1 mass%, and additionally, the standard deviation of particle diameters was higher than 0.3, indicating that the shapes of the particles were not uniform.

<Example 5: Preparation of Hyaluronic Acid>

A 200 L tank with a stirrer and a jacket was filled with 14 L (14 kg) of 8% hydrochloric acid and 53 L of ethanol, and the liquid was heated to a temperature of 70°C while being stirred. After the temperature reached 70°C, 5 kg of a starting raw material hyaluronic acid having an average molecular weight of 300,000 was loaded into the tank while the liquid was stirred. The liquid was stirred for 1 hour so that the hyaluronic acid fine powder was in a dispersion state while the liquid was heated so that the temperature of the aqueous ethanol containing hydrochloric acid (pH 0.2) was maintained at 70°C (solid phase decomposition step). Subsequently, the aqueous ethanol containing hydrochloric acid was removed by decantation, and 25 L of 2% hydrochloric acid (pH 1.0, 25 kg) was added to about 6.5 kg of the residual solid matter (the raw material hyaluronic acid moistened with 1.5 kg of ethanol) to prepare an acidic aqueous solution of the raw material hyaluronic acid (pH about 1) containing about 16 mass% of the raw material hyaluronic acid, which was subjected to hydrolysis at 70°C for 3 hours (liquid phase decomposition step). Further, the resultant liquid containing a hydrolysate was treated with an anion-exchange resin (trade name: DIAION or SEPABEADS, manufactured by Mitsubishi Chemical Corporation), and was pushed out of the column and diluted with 8 L of water, followed by removal of the resin using an 80 mesh filter. 2 kg of a 25% aqueous solution of sodium hydroxide was added thereto to adjust the pH to 4.0 or more and 6.5 or less. The resultant solution (the concentration of the hyaluronic acid: 12 mass%, in this example, the concentration of the hyaluronic acid was calculated on the assumption that the amount of the starting raw material hyaluronic acid added was the content of the hyaluronic acid) was dried by freeze-drying. Thus, 4.5 kg of a hyaluronic acid (yield: about 90%) was obtained as fine white powder. The average molecular weight of the raw material hyaluronic acid obtained in the solid phase decomposition step was separately measured and found to be about 10,000.

<Example 6: Preparation of Hyaluronic Acid>

A 200 L tank with a stirrer and a jacket was filled with 14 L (14 kg) of 8% hydrochloric acid and 53 L of ethanol, and the liquid was heated to a temperature of 70°C while being stirred. After the temperature reached 70°C, 5 kg of a starting raw material hyaluronic acid having an average molecular weight of 300,000 was loaded into the tank while the liquid was stirred. The liquid was stirred for 1 hour so that the hyaluronic acid fine powder was in a dispersion state while the liquid was heated so that the temperature of the aqueous ethanol containing hydrochloric acid (pH 0.2) was maintained at 70°C (solid phase decomposition step). Subsequently, the aqueous ethanol containing hydrochloric acid was removed by decantation, and 25 L of 2% hydrochloric acid (pH 1.0, 25 kg) was added to about 6.5 kg of the residual solid matter (the raw material hyaluronic acid moistened with 1.5 kg of ethanol) to prepare an acidic aqueous solution of the raw material hyaluronic acid (pH about 1) containing about 16 mass% of the raw material hyaluronic acid, which was subjected to hydrolysis at 70°C for 6 hours (liquid phase decomposition step). Further, the resultant liquid containing a hydrolysate was treated with an anion-exchange resin (trade name: DIAION or SEPABEADS, manufactured by Mitsubishi Chemical Corporation), and was pushed out of the column and diluted with 8 L of water, followed by removal of the resin using an 80 mesh filter. 2 kg of a 25% aqueous solution of sodium hydroxide was added thereto to adjust the pH to 4.0 or more and 6.5 or less. The resultant solution (the concentration of the hyaluronic acid: 12 mass%, in this example, the concentration of the hyaluronic acid was calculated on the assumption that the amount of the starting raw material hyaluronic acid added was the content of the hyaluronic acid) was dried by freeze-drying. Thus, 4.5 kg of a hyaluronic acid (yield: about 90%) was obtained as fine white powder. The average molecular weight of the raw material hyaluronic acid obtained in the solid phase decomposition step was separately measured and found to be about 10,000.

<Example 7: Preparation of Hyaluronic Acid>

A solution containing 22 mass% of a hyaluronic acid of Example 7 was obtained in the same manner as in Example 3 except that, in Example 3, the solution obtained after removal of the resin was directly used without being pushed out of the column and diluted with water. The solution was subjected to spray-drying to yield a hyaluronic acid of Example 7.

More specifically, a 2 L beaker was filled with 280 mL (280 g) of 8% hydrochloric acid and 1 L of ethanol, and the liquid was heated to a temperature of 70°C in a hot-water bath while being stirred. After the temperature reached 70°C, 50 g of a starting raw material hyaluronic acid having an average molecular weight of 300,000 was loaded into the beaker while the liquid was stirred. The liquid was stirred for 1 hour so that the hyaluronic acid fine powder was in a dispersion state while the liquid was heated so that the temperature of the aqueous ethanol containing hydrochloric acid (pH0.2) was maintained at 70°C (solid phase decomposition step). Subsequently, the aqueous ethanol containing hydrochloric acid was removed by decantation, and 150 mL of 2% hydrochloric acid (pH 1.0, 150 g) was added to about 60 g of the residual solid matter (the raw material hyaluronic acid moistened with 10 g of ethanol) to prepare an acidic aqueous solution of the raw material hyaluronic acid containing about 24 mass% of the raw material hyaluronic acid (pH 1.0), which was subjected to hydrolysis at 70°C for 5 hours (liquid phase decomposition step). Further, the resultant liquid containing a hydrolysate was treated with an anion-exchange resin (trade name: DIAION or SEPABEADS, manufactured by Mitsubishi Chemical Corporation), and the resin was removed using an 80 mesh filter. 20 g of a 25% aqueous solution of sodium hydroxide was added thereto to adjust the pH to 4.0 or more and 6.5 or less. The resultant solution (the concentration of the hyaluronic acid: 22 mass%, in this example, the concentration of the hyaluronic acid was calculated on the assumption that the amount of the starting raw material hyaluronic acid added was the content of the hyaluronic acid) was dried by spray-drying. Thus, 45 g of a hyaluronic acid (yield: about 90%) was obtained as fine white powder. The average molecular weight of the raw material hyaluronic acid obtained in the solid phase decomposition step was separately measured and found to be about 10,000.

<Example 8: Preparation of Hyaluronic Acid>

A solution containing 22 mass% of a hyaluronic acid of Example 8 was obtained in the same manner as in Example 7 except that, in Example 7, ethanol used in the solid phase decomposition step was replaced by methanol. The solution was subjected to spray-drying to yield a hyaluronic acid of Example 8.

The hyaluronic acid of Example 8 was equivalent to that of Example 7 in terms of the molecular weight, and the purity, the b value, the bulk density, the particle size distribution (including the average particle diameter and the standard deviation of particle diameters), the content of chlorine, the proportion of disaccharide, and the total proportion of disaccharide, tetrasaccharide, hexasaccharide, and octasaccharide of the hyaluronic acid.

<Comparative Example 9: Preparation of Hyaluronic Acid>

About 5 kg of a raw material hyaluronic acid having a molecular weight of 110,000 was moistened with 1 kg of ethanol, and the resultant was added to 25 L of 2% hydrochloric acid (pH 1.0, 25 kg) in a 50 L tank with a stirrer and a jacket for preparing an acidic aqueous solution of the raw material hyaluronic acid (pH about 1) containing about 16 mass% of the raw material hyaluronic acid, but the viscosity of the hyaluronic acid solution became much higher, and hence the solution could not be stirred.

<Comparative Example 10: Preparation of Hyaluronic Acid>

About 0.2 kg of a raw material hyaluronic acid having a molecular weight of 110,000 was moistened with 0.05 kg of ethanol, and the resultant was added to 25 L of 2% hydrochloric acid (pH 1.0, 25 kg) in a 50 L tank with a stirrer and a jacket to prepare an acidic aqueous solution of the raw material hyaluronic acid (pH about 1) containing about 0.8 mass% of the raw material hyaluronic acid. The resultant was retained at 70°C for 10 hours to be subjected to hydrolysis (liquid phase decomposition step) . Further, the resultant liquid containing a hydrolysate was treated with an anion-exchange resin (trade name: DIAION or SEPABEADS, manufactured by Mitsubishi Chemical Corporation), and was pushed out of the column and diluted with 8 L of water, followed by removal of the resin using an 80 mesh filter. 2 kg of a 25% aqueous solution of sodium hydroxide was added thereto to adjust the pH to 4.0 or more and 6.5 or less. When the resultant solution (the concentration of the hyaluronic acid: about 0.6 mass%, in this comparative example, the concentration of the hyaluronic acid was calculated on the assumption that the amount of the starting raw material hyaluronic acid added was the content of the hyaluronic acid) was dried by spray-drying (temperature of a heater: 180°C), the solution was turned into a fine mist, and hence the hyaluronic acid could not be collected.

The purity, thebvalue, the bulk density, the particle size distribution (including the average particle diameter and the standard deviation of particle diameters) of the hyaluronic acid, the content of chlorine, the proportion of disaccharide, and the total proportion of disaccharide, tetrasaccharide, hexasaccharide, and octasaccharide of the hyaluronic acids of Examples 2 to 7 and Comparative Examples 2 to 4, were measured in the same manner as in the case of the hyaluronic acid of Example 1. The results are shown in Table 1. The contents of chlorine and contents of sulfur in all of the hyaluronic acids of Examples 1 to 8 and Comparative Examples 2 to 4, 7, and 8 were 1 mass% or less.

[Table 1]

| | Example 1 | Comparative Example 2 | Example 2 | Example 3 | Example 4 | Comparative Example 3 | Comparative Example 4 | Comparative Example 7 | Comparative Example 8 | Example 5 | Example 6 | Example 7 |
|---|---|---|---|---|---|---|---|---|---|---|---|---|
| Bulk density (g/cm$^3$) | 0.27 | 0.07 | 0.28 | 0.43 | 0.33 | 0.01 | 0.10 | 0.60 | C.33 | 0.30 | 0.32 | C. 44 |
| Average molecular weight | 2,200 | 1,900 | 2,400 | 2,000 | 2,000 | 2,000 | 2,000 | 3,000 | 2,200 | 2,850 | 1,940 | 2,000 |
| Average particle diameter ($\mu$m) | 34.2 | 40.2 | 33.8 | 24.2 | 41.2 | 38.7 | - | 42.5 | 34.0 | 20.4 | 20.5 | 23.2 |
| Standard deviation of particle diameters | 0.290 | 0.456 | 0.310 | 0.234 | 0.472 | 0.447 | - | 0.438 | 0.389 | 0.447 | 0.390 | 0.222 |
| Content of chlorine (mass%) | 0.6 | 0.7 | 0.7 | 0.5 | 0.5 | 0.5 | 0.5 | 1.2 | 1.3 | 0.5 | 0.6 | 0.5 |
| Purity | 0.98 | 0.93 | 0.97 | 0.99 | 0.99 | 0.99 | 0.99 | 0.98 | C.98 | 0.98 | 0.99 | C.99 |
| b value | 2.2 | 2.8 | 2.0 | 2.7 | 2.7 | 2.7 | 2.7 | 3.2 | 2.0 | 2.2 | 2.1 | 2.7 |
| Proportion of monosaccharide (mass%) | 1.9 | 2.3 | 1.5 | 1.7 | 1.7 | 1.7 | 1.7 | 0.1 | 1.5 | 2.2 | 1.5 | 1.7 |
| Proportion of disaccharide | 11 | 12 | 9 | 9 | 9 | 9 | 9 | 3 | 10 | 8 | 5 | 9 |
| Proportion of octasaccharide (mass%) | 10 | 9 | 11 | 12 | 12 | 12 | 12 | 4 | 11 | 13 | 7 | 12 |
| Total proportion of disaccharide, tetrasaccharide, hexasaccharide, octasaccharide (mass%) | 45 | 48 | 43 | 44 | 44 | 44 | 44 | 10 | 44 | 44 | 33 | 44 |

EP 3 103 815 B1

As shown in Table 1, the hyaluronic acids of Examples 1 to 7 each have a bulk density of more than 0.25 g/cm$^3$, a small variation in particle diameter, a content of chlorine of 1 mass% or less, a content of sulfur of 1 mass% or less, and a proportion of disaccharide of 1 mass% or more and 30 mass% or less. In addition, it can be understood that the hyaluronic acids of Examples 1 to 7 each have a proportion of octasaccharide of 1 mass% or more and 30 mass% or less and a total proportion of disaccharide, tetrasaccharide, hexasaccharide, and octasaccharide of 5 mass% or more and 80 mass% or less.

In addition, 200 mg of each of the hyaluronic acids of Examples 1 to 7 and Comparative Examples 2 to 4, 7, and 8 was tableted to prepare tablets, and the tablets were compared for tableting adequacy. The hyaluronic acids of Examples 1 to 7 each had a high binding strength and high tableting adequacy compared to the hyaluronic acids of Comparative Examples 2 to 4, 7, and 8. In particular, the tablets containing the hyaluronic acids of Examples 3 to 7 each having a low content of chlorine had high tableting adequacy. In addition, the tablets containing the hyaluronic acids of Examples 1, 3, and 7 each having the smallest average particle diameter and a small standard deviation of particle diameters were hardly spread in air as powder, and were excellent in handling ability, in particular.

## Claims

1. A method of producing a hyaluronic acid and/or a salt thereof, the method comprising:

   preparing an acidic raw material hyaluronic acid solution that comprises an alcohol having 1, 2 or 3 carbon atoms, a raw material hyaluronic acid and/or a salt thereof having an average molecular weight of 3,500 or more and 100,000 or less, and an acid which can adjust the pH of the acidic raw material hyaluronic acid solution to an acidic pH;
   subjecting the raw material hyaluronic acid and/or the salt thereof to acid hydrolysis in the acidic raw material hyaluronic acid solution obtained in the preparing;
   treating an acidic solution that comprises a hydrolysate obtained through the acid hydrolysis with an anion-exchange resin; and
   subjecting the hydrolysate treated with the resin to spray-drying or freeze-drying,
   the acidic raw material hyaluronic acid solution comprising 15 mass% or more and 35 mass% or less of the raw material hyaluronic acid and/or the salt thereof,
   the acidic raw material hyaluronic acid solution comprising 5 parts by mass or more and 60 parts by mass or less of the alcohol having 1, 2 or 3 carbon atoms with respect to 100 parts by mass of the raw material hyaluronic acid and/or the salt thereof.

2. The method of producing a hyaluronic acid and/or a salt thereof according to claim 1, wherein:

   the spray-drying comprises subjecting a liquid comprising the hydrolysate to spray-drying; and
   a mass of the hydrolysate with respect to a mass of the liquid comprising the hydrolysate is 8 mass% or more and 35 mass% or less.

## Patentansprüche

1. Verfahren zur Herstellung einer Hyaluronsäure und/oder eines Salzes davon, wobei das Verfahren umfasst:

   Herstellen einer sauren Lösung von roher Hyaluronsäure, die einen Alkohol mit 1, 2 oder 3 Kohlenstoffatomen, eine rohe Hyaluronsäure und/oder ein Salz davon mit einem mittleren Molekulargewicht von 3.500 oder mehr und von 100.000 oder weniger und eine Säure, die den pH der sauren Lösung der rohen Hyaluronsäure auf einen sauren pH einstellen kann, umfasst;
   saure Hydrolyse der rohen Hyaluronsäure und/oder des Salzes davon in der beim Herstellen erhaltenen sauren Lösung von roher Hyaluronsäure;
   Behandeln einer sauren Lösung, die ein durch die saure Hydrolyse erhaltenes Hydrolysat umfasst, mit einem Anionenaustauscher-Harz; und
   Sprühtrocknen oder Gefriertrocknen des mit dem Harz behandelten Hydrolysats,
   wobei die saure Lösung von roher Hyaluronsäure 15 Masse-% oder mehr und 35 Masse-% oder weniger der rohen Hyaluronsäure und/oder des Salzes davon umfasst,
   wobei die saure Lösung von roher Hyaluronsäure 5 Masseteile oder mehr und 60 Masseteile oder weniger des Alkohols mit 1, 2 oder 3 Kohlenstoffatomen umfasst, bezogen auf 100 Masseteile der rohen Hyaluronsäure und/oder des Salzes davon.

2. Verfahren zur Herstellung einer Hyaluronsäure und/oder eines Salzes davon nach Anspruch 1, wobei:

das Sprühtrocknen Sprühtrocknen einer das Hydrolysat umfassenden Flüssigkeit umfasst; und
die Masse des Hydrolysats, bezogen auf die Masse der das Hydrolysat umfassenden Flüssigkeit, 8 Masse-% oder mehr und 35 Masse-% oder weniger beträgt.

**Revendications**

1. Procédé de production d'un acide hyaluronique et/ou d'un sel de celui-ci, le procédé comprenant les étapes consistant à :

préparer une solution d'acide hyaluronique en matériau acide brut qui comprend un alcool ayant 1, 2 ou 3 atomes de carbone, un acide hyaluronique en matériau brut et/ou un sel de celui-ci ayant un poids moléculaire moyen de 3 500 ou plus et de 100 000 ou moins, et un acide qui peut régler le pH de la solution d'acide hyaluronique en matériau acide brut à un pH acide ;
soumettre l'acide hyaluronique en matériau brut et/ou le sel de celui-ci à une hydrolyse acide dans la solution d'acide hyaluronique en matériau acide brut obtenue dans la préparation ;
traiter une solution acide qui comprend un hydrolysat obtenu par l'hydrolyse acide avec une résine échangeuse d'anions ; et
soumettre l'hydrolysat traité avec la résine à un séchage par pulvérisation ou à une lyophilisation,
la solution d'acide hyaluronique en matériau acide brut comprenant 15 % en masse ou plus et 35 % en masse ou moins de l'acide hyaluronique en matériau brut et/ou du sel de celui-ci,
la solution d'acide hyaluronique en matériau acide brut comprenant 5 parties en masse ou plus et 60 parties en masse ou moins de l'alcool ayant 1, 2 ou 3 atomes de carbone par rapport à 100 parties en masse de l'acide hyaluronique en matériau brut et/ou du sel de celui-ci.

2. Procédé de production d'un acide hyaluronique et/ou d'un sel de celui-ci selon la revendication 1, dans lequel :

le séchage par pulvérisation comprend la soumission d'un liquide comprenant l'hydrolysat à un séchage par pulvérisation ; et
une masse de l'hydrolysat par rapport à une masse du liquide comprenant l'hydrolysat est de 8 % en masse ou plus et de 35 % en masse ou moins.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2006265287 A
- JP 2009155486 A
- EP 2511302 A1

- JP 2013177608 A
- EP 1865002 A1
- EP 1992645 A1

### Non-patent literature cited in the description

- 3. Powder Property Determinations, 3.01 Determination of Bulk and Tapped Densities. the Japanese Pharmacopoeia
- General Tests, Processes and Apparatus of the Japanese Pharmacopoeia

- **TORVARD C LAURENT ; MARION RYAN ; ADOLPH PIETRUSZKIEWICZ.** Fractionation of hyaluronic Acid. *Biochemina et Biophysica Acta.,* 1960, vol. 42, 476-485
- **CHIKAKO YOMOTA.** Evaluation of Molecular Weights of Sodium Hyaluronate Preparations by SEC-MALLS. *Bull. Natl. Inst. Health Sci.,* 2003, vol. 121, 030-033